# EUROPÄISCHE PATENTSCHRIFT

(11) **EP 0 947 492 B1**
(45) Veröffentlichungstag und Bekanntmachung des Hinweises auf die Patenterteilung: **08.10.2003**
(21) Anmeldenummer: 99105903.1
(22) Anmeldetag: 24.03.1999
(51) Int. Cl.: C07C 45/51, C07C 47/20

(54) **Verfahren zur Herstellung von Citral**
Process for the preparation of citral
Procédé pour la préparation de citral

(30) Priorität: 30.03.1998 EP 98105757
(43) Veröffentlichungstag der Anmeldung: 06.10.1999
(73) Patentinhaber: F. HOFFMANN-LA ROCHE AG, 4070 Basel (CH)
(72) Erfinder: Bonrath, Werner, 79115 Freiburg (DE)

(56) Entgegenhaltungen:
- EP-A- 0 240 431
- GB-A- 1 204 754
- C.Y. LORBER AND J.A. OSBORN: "Cis-dioxomolybdenum (VI) complexes as new catalysts for the Meyer-Schuster Rearrangement" TETRAHEDRON LETTERS, Bd. 37, Nr. 6, 1996, Seiten 853-856, XP004030279
- ERMAN, M. B. ET AL: "The rearrangement of tertiary propargyl alcohols to.alpha.,.beta.-unsaturated aldehydes in the presence of polymeric organosilyl vanadates" TETRAHEDRON LETT. (1976), (34), 2981-4 CODEN: TELEAY, XP002107749

## Beschreibung

Die vorliegende Erfindung betrifft ein neues Verfahren zur Herstellung von Citral durch eine besondere katalysierte Umlagerung von Dehydrolinalool. Der α,β-ungesättigte Aldehyd Citral (E/Z-3,7-Dimethyl-2,6-octadienal, bestehend aus den Isomeren Geranial, d.h. E-Citral, und Neral, d.h. Z-Citral) ist bekanntlich ein wertvolles Zwischenprodukt für die Synthese von Geruchsstoffen, Terpinoiden und Vitaminen.

α,β-Ungesättigte Carbonylverbindungen sind im allgemeinen wichtige Zwischenprodukte für die Herstellung von Geruchsstoffen, Vitaminen und Carotinoiden [siehe beispielsweise Chem. Ztg. 97, 23-28 (1973) und Kap. VI ("Total Syntheses") in "Carotenoids", Ed. Otto Isler, Birkhäuser Verlag Basel und Stuttgart, 1971]. Ihre Herstellung durch säurekatalysierte Umlagerung von α-Alkinolen wurde bereits in den zwanziger Jahren von K. H. Meyer und K. Schuster [Ber. deutsch. Chem. Ges. 55, 819-823 (1922)] und H. Rupe und E. Kambli [Helv. Chim. Acta 9, 672 (1926)] beschrieben; die Isomerisierung sekundärer oder tertiärer α-Alkinole zu α,β-ungesättigten Carbonylverbindungen ist auch allgemein als Meyer-Schuster- bzw. Rupe-Kambli-Umlagerung bekannt geworden. Handelt es sich um die Umlagerung einer Carbonylverbindung mit einer endständigen Alkinylgruppe, werden Aldehyde erhalten, andernfalls sind Ketone die Umlagerungsprodukte: 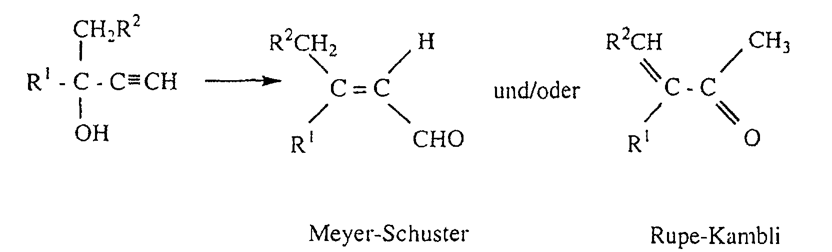 worin R¹ und R² jeweils Wasserstoff oder einen aliphatischen oder aromatischen Rest bedeuten. Neben Citral sind auch die ebenfalls α,β-ungesättigten Aldehyde Citronellal und Hydroxycitronellal von besonderem industriellem Interesse, und zwar als Zwischenprodukte für die Herstellung von Geruchsstoffen, Terpinoiden und Vitaminen; Citral selber kann jeweils in mehreren Verfahrensstufen in die wichtigen Ausgangsmaterialien zur Herstellung von d,l-α-Tocopherol (Vitamin E) und Vitamin A, Isophytol bzw. β-Ionon, übergeführt werden [siehe beispielsweise "Vitamine I, Fettlösliche Vitamine", Ed. Otto Isler und Georg Brubacher, Georg Thieme Verlag Stuttgart, New York 1982, das Kapitel VI "Total Syntheses" in "Carotenoids" (Birkhäuser Verlag 1971) und die darin erwähnten Literaturstellen.

Die durch Silber- oder Kupferionen katalysierte Umlagerung von Dehydrolinalylacetat liefert nach G. Saucy et al. [Helv. Chim. Acta 42, 1945-1955 (1959)] in Abhängigkeit von den Reaktionsbedingungen ein Gemisch aus "Allenacetat" (1-Acetoxy-3,7-dimethyl-octa-1,2,6-trien) und "Diacetat" (1,1-Diacetoxy-3,7-dimethyl-octa-2,6-dien), das sich zu Citral hydrolysieren lässt:

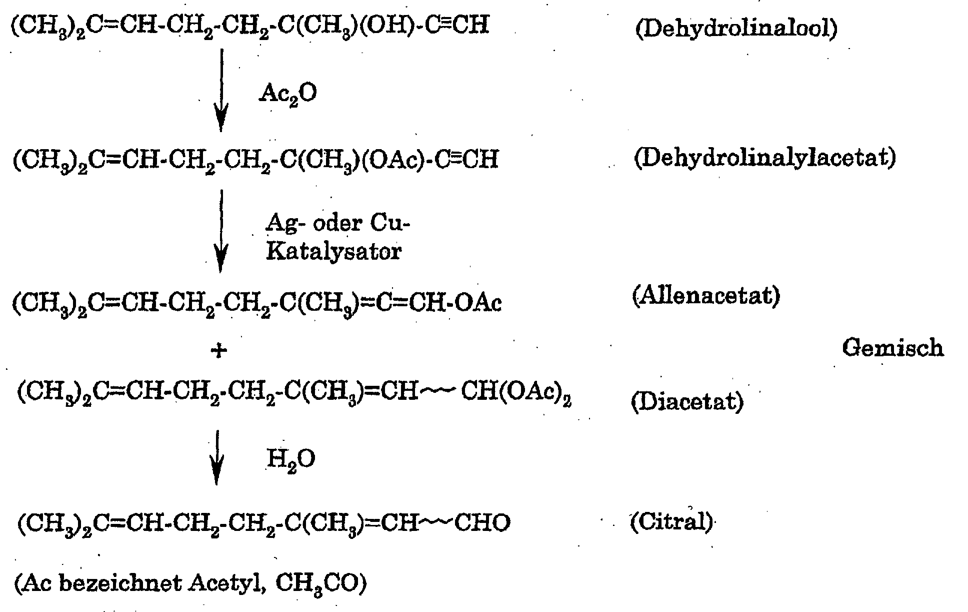

Diese Umlagerung von Dehydrolinalylacetat ist als Saucy-Marbet-Umlagerung bekannt. Allerdings kann Dehydrolinalool unter Verwendung eines Alkyl-, Cycloalkyl- oder Arylorthovanadats oder eines anderen Vanadium-Katalysators unmittelbar in Citral übergeführt werden (UK-Patentschrift 1.204.754). Nachteilig an der unmittelbaren Ueberführung sind allerdings die niedrige Ausbeute unter Berücksichtigung der Dehydrolinalool-Umsatzes maximal etwa 30% sowie die Bildung dunkler Niederschläge, die zur Zersetzung der Reaktionslösung führen. Wesentlich selektiver und effizienter erfolgt die direkte Umlagerung von Dehydrolinalool unter Verwendung von Tris(triphenylsilyl)vanadiumoxid bei etwa 140°C [Chimia 27, 383 (1973) sowie Helv. Chim. Acta 59, 1233-1243 (1976)]. Hierbei werden in Paraffinöl als Lösungsmittel Ausbeuten von etwa 78% erzielt.

Weitere Veröffentlichungen der unmittelbaren Umlagerung von Dehydrolinalool zu Citral unter Verwendung von vanadiumhaltigen Katalysatoren beinhalten die Verwendung von Polyboroxyvanadoxydiphenylsilan und von Polysilylvanadaten als Katalysatoren [Tschechoslowakische Patentschrift CS 264.720/Chem. Abs. 114, 122769a (1991) bzw. Mendeleev Commun. 1994, 89]. Während im ersten Verfahren die erzielte etwa 70%ige Ausbeute kommerziell zu niedrig ist, kann man mit dem zweiten Verfahren eine 80%ige Ausbeute erzielen.

Ein weiterer Katalysator für die unmittelbare Umlagerung von α-Alkinolen, wie beispielsweise Dehydrolinalool, zu α,β-ungesättigten Carbonylverbindungen besteht aus der Kombination einer Titanverbindung, z.B. Titantetrachlorid oder -tetrabutoxid, mit einem Kupfer- oder Silberhalogenid [Tetr. Lett. 29, 6253-6256 (1988) und Europäische Patentpublikation 0 240 431 A]. Nachteilig an diesem Verfahren ist allerdings die Verwendung von Kupferverbindungen. Zudem ist auch in diesem Fall die erzielte etwa 64%ige Ausbeute an Citral unzufriedenstellend.

Eine interessante Variante der oben erwähnten Meyer-Schuster-Umlagerung wurde kürzlich von C.Y. Lorber und J.A. Osborn in Tetr. Lett. 37, 853-856 (1996) beschrieben; es handelt sich dabei um die Umlagerung von Methylbutinol zu Prenal unter Verwendung eines Molybdän-Katalysators. Hierbei wird in ortho-Dichlorbenzen als Lösungsmittel Methylbutinol in Gegenwart des Katalysatorsystems Molybdänylacetylacetonat, Dibutylsulfoxid und 4-tert. Butylbenzoesäure zu Prenal umgelagert. Obwohl die Ausbeute und Selektivität dieser Umlagerung mit 90% bzw- 99% angegeben sind, wurde das Prenal nicht aus dem Reaktionsgemisch isoliert, sondern die angebliche Ausbeute durch gaschromatographische Analyse des rohen Produktes eruiert. Vermutlich war es schwierig, das Reaktionsgemisch aufzuarbeiten, um Prenal zu isolieren.

L.A. Kheifits und Mitarbeiter fanden, dass Dehydrolinalool bei 170°C in 14 Stunden Reaktionszeit lediglich in 28%iger Ausbeute in Citral und in 12%iger Ausbeute in 2-Hydroxymethyl-1-methyl-3-isopropenylcyclopent-1-en übergeführt werden konnte, wenn als Katalysator ein aus Natriummolybdänat und Diphenyldichlorsilan hergestelltes Molybdän-Polymer für die Umlagerung verwendet wurde [Tetr. Lett. 34, 2981-2984 (1976)].

Aus den obigen Ausführungen ist ersichtlich, dass die vorbekannten Verfahren zur katalysierten Umlagerung von α-Alkinolen, z.B. Dehydrolinalool, zu α,β-ungesättigten Aldehyden, z.B. Citral, gewichtige Nachteile aufweisen.

Der vorliegenden Erfindung liegt nun die Aufgabe zugrunde, ein Verfahren zur Umlagerung von Dehydrolinalool zu Citral unter Einwirkung eines Katalysators bereitzustellen, das die Nachteile der vorbekannten Verfahren nicht oder zumindest viel weniger aufweist. Diese Aufgabe wird überraschend gut gelöst, indem man nicht nur die bekannten Molybdänverbindung Molybdänylacetylacetonat [auch als Dioxomolybdän(VI)acetylacetonat bekannt] oder ein Molybdänylhalogenid als Katalysator verwendet, sondern diese Verbindung als Bestandteil eines bestimmten Katalysatorsystems einsetzt und ansonsten die Umlagerung unter gewissen Reaktionsbedingungen durchführt.

Bei dem erfindungsgemässen Verfahren handelt es sich um ein Verfahren zur Herstellung von Citral durch katalysierte Umlagerung von Dehydrolinalool zu Citral, das dadurch gekennzeichnet ist, dass man die Umlagerung in Gegenwart einer Molybdänverbindung der allgemeinen Formel

MoO₂X₂ I

worin X einen Acetylacetonat- oder Halogenidion bedeutet, und eines Dialkyl- oder Diarylsulfoxids als Katalysatorsystem, in Gegenwart einer organischen Säure mit einem pK-Wert im Bereich von 4,0 bis 6,5 sowie in einem apolaren aprotischen organischen Lösungsmittel durchführt.

Die Molybdänverbindung der Formel I, d.h. Molybdänylacetylacetonat (konventionell als MoO₂acac₂ bezeichnet) oder ein Molybdänylhalogenid der Formel MoO₂(Hal)₂ [X = Hall, worin Hal Chlor oder Brom bedeutet, ist jeweils eine im Handel leicht erhältliche bekannte Verbindung. Das Molybdänylhalogenid ist vorzugsweise Molybdänylchlorid, MoO₂Cl₂. Die bevorzugte Molybdänverbindung der Formel I ist allerdings Molybdänylacetylacetonat.

Bei dem ebenfalls im Katalysatorsystem vorhandenen Dialkyl- oder Diarylsulfoxid handelt es sich insbesondere um ein Dialkylsulfoxid, dessen Alkylgruppen jeweils geradkettig oder verzweigt sind und bis zu 8 Kohlenstoffatome enthalten, bzw. um ein Diarylsulfoxid, dessen Arylgruppen jeweils gegebenenfalls substituierte Phenylgruppen sind. Im letzteren Fall können die allfällig vorhandenen Substituenten insbesondere C₁₋₄-Alkylgruppen sein, wobei die Phenylgruppe jeweils einfach oder mehrfach alkylsubstituiert ist. Beispiele beider Typen von Sulfoxiden sind Dimethylsulfoxid und Dibutylsulfoxid bzw. Diphenylsulfoxid und Di(p-tolyl)sulfoxid. Vorzugsweise wird als Sulfoxid das Dimethylsulfoxid verwendet.

Als organische Säuren mit einem pK-Wert im Bereich von 4,0 bis 6,5 kommen u.a. in Frage gegebenenfalls halogenierte, gesättigte und ungesättigte aliphatische Carbonsäuren, z.B. Essigsäure (pK-Wert 4,74), Propionsäure (4,87), Chlorpropionsäure (3,98) und Pivalinsäure (5,01) bzw. Acrylsäure (4,25); Alkandicarbonsäuren, z.B. Adipinsäure (4,40); arylsubstituierte Alkancarbonsäuren, z.B. Phenylessigsäure (4,25); sowie aromatische Carbonsäuren, z.B. Benzoesäure (4,19) und 4-tert.Butyl-benzoesäure (6,50). Vorzugsweise verwendet man eine organische Säure mit einem pK-Wert im Bereich von etwa 4,25 bis etwa 6,5, insbesondere Phenylessigsäure mit dem pK-Wert 4,25.

Als Lösungsmittel können im Rahmen der vorliegenden Erfindung im allgemeinen apolare aprotische organische Lösungsmittel, insbesondere aliphatische, cyclische und aromatische Kohlenwasserstoffe, wie beispielsweise C₇₋₁₀-Alkane, C₅₋₇-Cycloalkane, Benzen, Toluen und Naphthalen sowie Gemische solcher Lösungsmittel untereinander, z.B. Paraffinöl (Gemisch gesättigter aliphatischer Kohlenwasserstoffe) verwenden werden. Toluen stellt ein besonders bevorzugtes Lösungsmittel dar.

Die Umlagerung erfolgt zweckmässigerweise bei Temperaturen im Bereich von 80°C bis 140°C, vorzugsweise bei Temperaturen von etwa 90°C bis etwa 120°C.

Die Menge Molybdänverbindung der Formel I beträgt zweckmässigerweise etwa 0,1 - 8 Mol-% bezogen auf die Menge eingesetzten Dehydrolinalools (Edukt). Diese Menge beträgt vorzugsweise etwa 1-7 Mol-%, ganz bevorzugt etwa 3-5 Mol-%.

Darüber hinaus beträgt das Gewichtsverhältnis Dialkyl- oder Diarylsulfoxid zu Edukt zweckmässigerweise 0,2:1 bis 1:1; das Gewichtsverhältnis Säure zu Edukt zweckmässigerweise 0,02:1 bis 0,1:1, vorzugsweise 0,04:1 bis 0,07:1, besonders bevorzugt 0,05:1; und das Gewichtsverhältnis Lösungsmittel zu Edukt zweckmässigerweise 5:1 bis 15:1, vorzugsweise 7:1 bis 10:1.

Das erfindungsgemässe Verfahren kann arbeitstechnisch sehr einfach durchgeführt werden, indem man das Edukt, das Katalysatorsystem (Molybdänverbindung der Formel I sowie Dialkyl- oder Diarylsulfoxid) und die organische Säure zum Lösungsmittel gibt und das Reaktionsgemisch, welches normalerweise wegen der unterschiedlichen Löslichkeiten der Reaktionsteilnehmer aus einer Suspension besteht, auf die Reaktionstemperatur erhitzt. Die Reihenfolge der Zugabe ist nicht kritisch, so dass beispielsweise die Säure oder das Sulfoxid zuletzt zugegeben werden kann. Zur Reaktionskontrolle können Proben entnommen und nach bekannten Methoden, z.B. Dünnschichtchromatographie oder Gaschromatographie, analysiert werden. Nach beendeter Reaktion, wobei die Reaktionsdauer normalerweise bis zu etwa 20 Stunden, vorzugsweise bis zu etwa 7 Stunden, beträgt, kann die Aufarbeitung durch gängige Verfahren der organischen Chemie erfolgen. Typischerweise wird das Gemisch filtriert und das Produkt Citral aus dem Filtrat durch Eindampfen isoliert. Zur Reinigung des Produktes kann die Rohware beispielsweise destilliert werden.

Das erfindungsgemässe Verfahren wird durch die nachfolgenden Beispiele veranschaulicht:

### Beispiel 1

### Umlagerung in verschiedenen Lösungsmitteln

In einem mit Thermometer, Rührer und Kühler versehenen 100 ml Sulfierkolben wurden 6,02 g (39,62 mmol) Dehydrolinalool (nachfolgend "DLL"), 2,31 g (29,67 mmol) Dimethylsulfoxid (nachfolgend "DMSO"), 0,65 g (1,99 mmol) Molybdänylacetylacetonat (nachfolgend "MoO₂acac₂") und 2,60 g (14,58 mmol) 4-tert.Butylbenzoesäure in 50 ml Lösungsmittel vorgelegt. Anschliessend wurde auf 100°C erwärmt. Hierbei erfolgte je nach Verfahrensvariante ein Farbwechsel des Reaktionsgemisches nach dunkelblau oder dunkelgrünblau. Zur Reaktionskontrolle wurden Proben entnommen und mittels Dünnschichtchromatographie (DC) oder Gaschromatographie (GC) analysiert. Nach beendeter Reaktion wurde aufgearbeitet, indem über wenig Kieselgel filtriert und anschliessend unter vermindertem Druck eingeengt wurde. Die Gehaltsbestimmung erfolgte durch GC mit internem Standard. Es wurden die in der nachfolgenden Tabelle 1 zusammengefassten Ergebnisse erhalten:

**Tabelle 1**

| Lösungsmittel | Ausbeute an Citral | Verbleibendes DLL |
|---|---|---|
| Toluen | 88% | 0% |
| Paraffinöl | 80% | 10% |

### Beispiel 2

### Umlagerung in Gegenwart verschiedener Säuren

In einem mit Thermometer, Rührer und Kühler versehenen 100 ml Sulfierkolben wurden 6,02 g (39,62 mmol) DLL, 2,31 g (29,67 mmol) DMSO und 0,65 g (1,99 mmol) MoO₂acac₂ in 50 ml Toluen vorgelegt und mit jeweils 14,58 mmol Säure versetzt. Anschliessend wurde auf 100°C erwärmt und nach beendeter Reaktion (DC- und GC-Kontrolle) wie in Beispiel 1 beschrieben aufgearbeitet. Es wurden die in der nachfolgenden Tabelle 2 zusammengefassten Ergebnisse erhalten:

**Tabelle 2**

| Säure | Ausbeute an: | | | Verbleibendes DLL |
|---|---|---|---|---|
| | Neral (Z-Citral) | Geranial (E-Citral) | Citral (Total) | |
| Stearinsäure | 30,84% | 36,98% | 67,82% | 14,12% |
| | | | | |
| Essigsäure | 23,16% | 27,74% | 50,90% | 26,30% |
| | | | | |
| Benzoesäure | 29,59% | 47,48% | 77,07% | 0% |
| | | | | |
| Propionsäure | 28,99% | 33,97% | 62,97% | 28,02% |
| | | | | |
| Pivalinsäure | 36,26% | 44,55% | 80,81% | 0% |
| | | | | |
| Acrylsäure | 37,00% | 46,66% | 83,65% | 0% |
| | | | | |
| Adipinsäure | 39,07% | 45,23% | 84,30% | 0% |
| | | | | |
| Phenylessigsäure | 38,92% | 47,85% | 86,77% | 6,83% |
| | | | | |
| 4-tert.Butylbenzoesäure | 39,35% | 48,27% | 87,63% | 1,10% |

### Beispiel 3

### Ermittlung eines typischen Reaktionsverlaufes

In einem mit Thermometer, Rührer und Kühler versehenen 100 ml Sulfierkolben wurden 6,02 g (39,62 mmol) DLL, 2,32 g (29,67 mmol) DMSO, 0,65 g (1,983 mmol) MoO₂acac₂ und 1,99 g (14,98 mmol) Phenylessigsäure in 50 ml Toluen auf 100°C erwärmt. Während 23,5 Stunden wurde bei dieser Temperatur gerührt und in bestimmten Zeitintervallen Proben entnommen und mittels GC bzw. DC analysiert. Zur Gaschromatographie wurden 700 µl Reaktionslösung entnommen und durch kurze Filtration vom Katalysator befreit. Diese Probe wurde eingewogen und mittels GC analysiert. Es wurden die in der nachfolgenden Tabelle 3 zusammengefassten Ausbeuten erhalten:

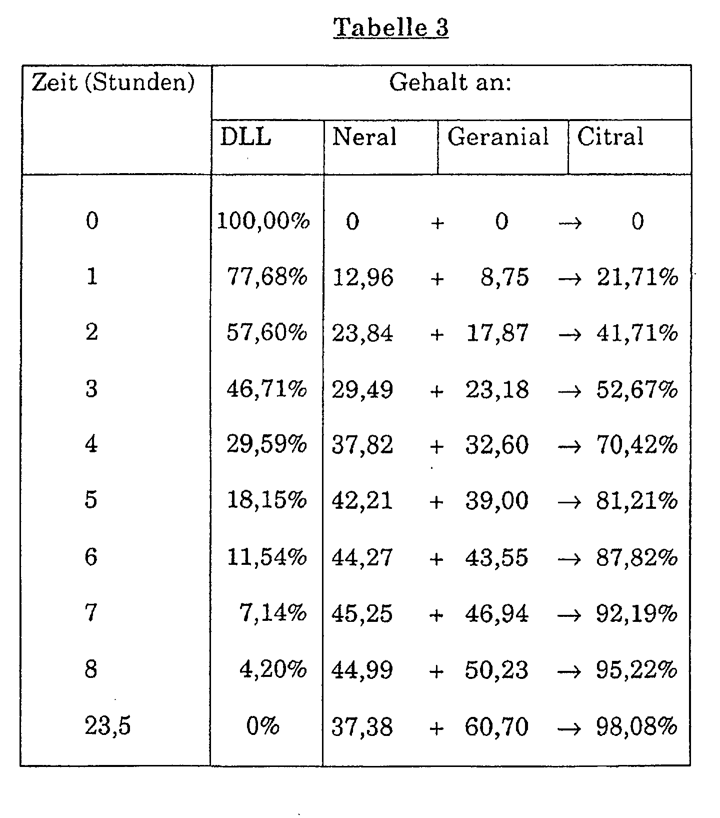

### Beispiel 4

In einem mit Rührer, Thermometer und Kühler versehenen 100 ml Sulfierkolben wurden 6,02 g (39,62 mmol) DLL, 2,31 g (29,67 mmol) DMSO, 1,99 g (14,88 mmol) Phenylessigsäure und 0,65 g (1,983 mmol) MoO₂acac₂ in 50 ml Toluen auf 100°C erwärmt. Nach 17 Stunden Reaktionszeit wurde auf Raumtemperatur gekühlt, über 10 g Kieselgel filtriert und mit 100 ml Toluen nachgewaschen. Das Filtrat wurde bei 25 mbar (2,5 KPa) und 40°C bis zur Gewichtskonstanz aufkonzentriert. Erhalten wurden 11,89 g einer gelbbraunen Rohware. Die Gehaltsbestimmung erfolgte mittels GC. Gefunden wurden:

| | | |
|---|---|---|
| Gehalt | Neral 21,08% | Geranial 22,55% |
| Ausbeute | Neral 2,51 g (41,63%) | Geranial 2,68 g (44,54%) |

Hieraus ergibt sich eine Ausbeute an Citral (E+Z) von 5,19 g (86,17%). Weiterhin wurden noch 2,08% (0,25 g) nicht umgesetztes DLL festgestellt. Von den umgesetzten 5,77 g DLL wurden demach 89,95% in Citral umgelagert.

### Beispiel 5

In einem mit Rührer, Thermometer und Kühler versehenen 100 ml Sulfierkolben wurden 6,02 g (39,62 mmol) DLL, 2,31 g (29,67 mmol) DMSO, 2,60 g (14,58 mmol) 4-tert.Butylbenzoesäure und 0,65 g (1,983 mmol) MoO₂acac₂ in 50 ml Toluen auf 100°C erwärmt. Nach 17 Stunden Reaktionszeit wurde auf Raumtemperatur gekühlt, über 10 g Kieselgel filtriert und mit 100 ml Toluen nachgewaschen. Das Filtrat wurde bei 25 mbar (2,5 KPa) und 40°C bis zur Gewichtskonstanz aufkonzentriert. Erhalten wurden 11,05 g einer gelbbraunen Rohware. Die Gehaltsbestimmung erfolgte mittels GC. Gefunden wurden:

| | | |
|---|---|---|
| Gehalt | Neral 21,44% | Geranial 26,30% |
| Ausbeute | Neral 2,37 g (39,35%) | Geranial 2,91 g (48,27%) |

Hieraus ergibt sich eine Ausbeute an Citral (E+Z) von 5,27 g (87,63%). Weiterhin wurden noch 1,10% (0,12 g) nicht umgesetztes DLL festgestellt. Von den umgesetzten 5,90 g DLL wurden demach 89,32% in Citral umgelagert.

### Beispiel 6

In einem mit Rührer, Thermometer und Kühler versehenen 100 ml Sulfierkolben wurden 6,02 g (39,62 mmol) DLL, 2,31 g (29,67 mmol) DMSO, 1,78 g (14,58 mmol) Benzoesäure und 0,65 g (1,983 mmol) MoO₂acac₂ in 50 ml Toluen auf 100°C erwärmt. Nach 17 Stunden Reaktionszeit wurde auf Raumtemperatur gekühlt, über 10 g Kieselgel filtriert und mit 100 ml Toluen nachgewaschen. Das Filtrat wurde bei 25 mbar (2,5 KPa) und 40°C bis zur Gewichtskonstanz aufkonzentriert. Erhalten wurden 9,35 g einer gelbbraunen Rohware. Die Gehaltsbestimmung erfolgte mittels GC. Gefunden wurden:

| | | |
|---|---|---|
| Gehalt | Neral 19,05% | Geranial 30,09% |
| Ausbeute | Neral 1,78 g (29,59%) | Geranial 2,86 g (47,48%) |

Hieraus ergibt sich eine Ausbeute an Citral (E+Z) von 4,63 g (77,07%). Es wurde kein DLL festgestellt.

### Beispiel 7

In einem mit Rührer, Thermometer und Kühler versehenen 100 ml Sulfierkolben wurden 6,02 g (39,62 mmol) DLL, 2,31 g (29,67 mmol) DMSO, 2,13 g (14,58 mmol) Adipinsäure und 0,65 g (1,983 mmol) MoO₂acac₂ in 50 ml Toluen auf 100°C erwärmt. Nach 17 Stunden Reaktionszeit wurde auf Raumtemperatur gekühlt, über 10 g Kieselgel filtriert und mit 100 ml Toluen nachgewaschen. Das Filtrat wurde bei 25 mbar (2,5 KPa) und 40°C bis zur Gewichtskonstanz aufkonzentriert. Erhalten wurden 6,41 g einer gelbbraunen Rohware. Die Gehaltsbestimmung erfolgte mittels GC. Gefunden wurden:

| | | |
|---|---|---|
| Gehalt | Neral 36,69% | Geranial 42,48% |
| Ausbeute | Neral 2,35 g (39,07%) | Geranial 2,72 g (45,23%) |

Hieraus ergibt sich eine Ausbeute an Citral (E+Z) von 5,07 g (84,30%). Es wurde kein DLL festgestellt.

## Patentansprüche

1. Verfahren zur Herstellung von Citral durch katalysierte Umlagerung von Dehydrolinalool zu Citral, **dadurch gekennzeichnet, dass** man die Umlagerung in Gegenwart einer Molybdänverbindung der allgemeinen Formel
MoO₂X₂ I
worin X einen Acetylacetonat- oder Halogenidion bedeutet, und eines Dialkyl- oder Diarylsulfoxids als Katalysatorsystem, in Gegenwart einer organischen Säure mit einem pK-Wert im Bereich von 4,0 bis 6,5 sowie in einem apolaren aprotischen organischen Lösungsmittel durchführt.

2. Verfahren nach Anspruch 1, **dadurch gekennzeichnet, dass** die Molybdänverbindung der Formel I Molybdänylacetylacetonat oder Molybdänylchlorid, vorzugsweise Molybdänylacetylacetonat, ist.

3. Verfahren nach Anspruch 1 oder 2, **dadurch gekennzeichnet, dass** das Dialkyl- oder Diarylsulfoxid Dimethylsulfoxid oder Dibutylsulfoxid bzw. Diphenylsulfoxid oder Di(p-tolyl)sulfoxid, vorzugsweise Dimethylsulfoxid, ist.

4. Verfahren nach einem der Ansprüche 1 bis 3, **dadurch gekennzeichnet, dass** als organische Säure eine gegebenenfalls halogenierte, gesättigte oder ungesättigte aliphatische Carbonsäure, eine Alkandicarbonsäure, eine arylsubstituierte Alkancarbonsäure oder eine aromatische Carbonsäure verwendet wird.

5. Verfahren nach Anspruch 4, **dadurch gekennzeichnet, dass** die organische Säure Essigsäure, Propionsäure, Chlorpropionsäure, Pivalinsäure, Acrylsäure, Adipinsäure, Phenylessigsäure, Benzoesäure oder 4-tert.Butylbenzoesäure, vorzugsweise Phenylessigsäure, ist.

6. Verfahren nach einem der Ansprüche 1 bis 5, **dadurch gekennzeichnet, dass** als Lösungsmittel ein aliphatischer Kohlenwasserstoff, ein cyclischer Kohlenwasserstoff oder ein aromatischer Kohlenwasserstoff, oder ein Gemisch solcher Lösungsmittel untereinander, verwendet wird.

7. Verfahren nach Anspruch 6, **dadurch gekennzeichnet, dass** das Lösungsmittel ein C₇₋₁₀-Alkan, ein C₅₋₇-Cycloalkan, Benzen, Toluen, Naphthalen oder Paraffinöl, vorzugsweise Toluen, ist.

8. Verfahren nach einem der Ansprüche 1 bis 7, **dadurch gekennzeichnet, dass** die Umlagerung bei Temperaturen im Bereich von 80°C bis 140°C, vorzugsweise bei Temperaturen von 90°C bis 120°C, erfolgt.

9. Verfahren nach einem der Ansprüche 1 bis 8, **dadurch gekennzeichnet, dass** die Menge Molybdänverbindung der Formel I 0,1 - 8 Mol-% bezogen auf die Menge eingesetzten Dehydrolinalools, vorzugsweise 1-7 Mol-%, ganz bevorzugt 3-5 Mol-%, beträgt.

10. Verfahren nach einem der Ansprüche 1 bis 9, **dadurch gekennzeichnet, dass** das Gewichtsverhältnis Dialkyl- oder Diarylsulfoxid zu Dehydrolinalool (Edukt) zweckmässigerweise 0,2:1 bis 1:1; das Gewichtsverhältnis Säure zu Edukt zweckmässigerweise 0,02:1 bis 0,1:1, vorzugsweise 0,04:1 bis 0,07:1, besonders bevorzugt 0,05:1; und das Gewichtsverhältnis Lösungsmittel zu Edukt etwa 5:1 bis etwa 15:1, vorzugsweise 7:1 bis 10:1, beträgt.

## Claims

1. A process for the manufacture of citral by the catalytic rearrangement of dehydrolinalool to citral, which process comprises carrying out the rearrangement in the presence of a molybdenum compound of the general formula
MoO₂X₂ I
wherein X signifies an acetylacetonate or halide ion,
and a dialkyl or diaryl sulphoxide as the catalyst system, in the presence of an organic acid having a pK value in the range of 4.0 to 6.5 and in an apolar aprotic organic solvent.

2. A process according to claim 1, wherein the molybdenum compound of formula I is molybdenyl acetylacetonate or molybdenyl chloride, preferably molybdenyl acetylacetonate.

3. A process according to claim 1 or claim 2, wherein the dialkyl or diaryl sulphoxide is dimethyl sulphoxide or dibutyl sulphoxide or, respectively, diphenyl sulphoxide or di(p-tolyl) sulphoxide, preferably dimethyl sulphoxide.

4. A process according to any one of claims 1 to 3, wherein an optionally halogenated, saturated or unsaturated aliphatic carboxylic acid, an alkanedicarboxylic acid, an aryl-substituted alkanecarboxylic acid or an aromatic carboxylic acid is used as the organic acid.

5. A process according to claim 4, wherein the organic acid is acetic acid, propionic acid, chloropropionic acid, pivalic acid, acrylic acid, adipic acid, phenylacetic acid, benzoic acid or 4-tert.butyl-benzoic acid, preferably phenylacetic acid.

6. A process according to any one of claims 1 to 5, wherein an aliphatic hydrocarbon, a cyclic hydrocarbon or an aromatic hydrocarbon or a mixture of such solvents with one another is used as the solvent.

7. A process according to claim 6, wherein the solvent is a C₇₋₁₀-alkane, a C₅₋₇-cycloalkane, benzene, toluene, naphthalene or paraffin oil, preferably toluene.

8. A process according to any one of claims 1 to 7, wherein the rearrangement is effected at temperatures in the range of 80°C to 140°C, preferably at temperatures of 90°C to 120°C.

9. A process according to any one of claims 1 to 8, wherein the amount of molybdenum compound of formula I is 0.1-8 mol% based on the amount of dehydrolinalool employed, preferably 1-7 mol%, particularly 3-5 mol%.

10. A process according to any one of claims 1 to 9, wherein the weight ratio of dialkyl or diaryl sulphoxide to dehydrolinalool (educt) is conveniently 0.2:1 to 1:1; the weight ratio of acid to educt is conveniently 0.02:1 to 0.1:1, preferably 0.04:1 to 0.07:1, particularly 0.05:1; and the weight ratio of solvent to educt is 5:1 to 15:1, preferably 7:1 to 10:1.

## Revendications

1. Procédé pour la préparation de citral par un réarrangement catalytique de déhydrolinalool en citral, **caractérisé en ce que** l'on mène le réarrangement en présence d'un composé à base de molybdène de formule générale
MoO₂X₂ I
dans laquelle X représente un ion acétylacétonate ou halogénure, d'un dialkyl- ou diarylsulfoxyde en tant que système catalytique, en présence d'un acide organique avec une valeur de pK dans la plage allant de 4,0 à 6,5 ainsi qu'un solvant organique aprotique apolaire.

2. Procédé selon la revendication 1, **caractérisé en ce que** le composé à base de molybdène de formule I est de l'acétylacétonate de molybdènyle ou du chlorure de molybdènyle, de préférence de l'acétylacétonate de molybdènyle.

3. Procédé selon la revendication 1 ou la revendication 2, **caractérisé en ce que** le dialkyl- ou diarylsulfoxyde est du diméthylsulfoxyde ou du dibutylsulfoxyde ou bien du diphénylsulfoxyde ou du di(p-tolyl)sulfoxyde, de préférence du diméthylsulfoxyde.

4. Procédé selon l'une quelconque des revendications 1 à 3, **caractérisé en ce qu'**est utilisé en tant qu'acide organique un acide carboxylique aliphatique, saturé ou insaturé, le cas échéant halogéné, un acide alcanedioïque, un acide alcanoïque substitué par un aryle ou un acide carboxylique aromatique.

5. Procédé selon la revendication 4, **caractérisé en ce que** l'acide organique est de l'acide acétique, de l'acide propionique, de l'acide chloroprioponique, de l'acide pivalique, de l'acide acrylique, de l'acide adipique, de l'acide phénylacétique, de l'acide benzoïque ou de l'acide 4-tert.butylbenzoique, de préférence de l'acide phénylacétique.

6. Procédé selon l'une quelconque des revendications 1 à 5, **caractérisé en ce qu'**est utilisé en tant que solvant un hydrocarbure aliphatique, un hydrocarbure cyclique ou un hydrocarbure aromatique, ou un mélange de tels solvants entre eux.

7. Procédé selon la revendication 6, **caractérisé en ce que** le solvant est un alcane en C₇₋C₁₀, un cycloalcane en C₅₋C₇, du benzène, du toluène, du naphtalène ou de l'huile de paraffine, de préférence du toluène.

8. Procédé selon l'une quelconque des revendications 1 à 7, **caractérisé en ce que** le réarrangement a lieu à des températures dans la plage allant de 80 °C à 140 °C, de préférence à des températures de 90 °C à 120 °C.

9. Procédé selon l'une quelconque des revendications 1 à 8, **caractérisé en ce que** la quantité de composé à base de molybdène de formule I s'élève de 0,1 à 8 % en moles rapportés à la quantité de déhydrolinalool mis en jeu, de préférence de 1 à 7 % en moles, complètement préféré de 3 à 5 % en moles.

10. Procédé selon l'une quelconque des revendications 1 à 9, **caractérisé en ce que** le rapport en poids du dialkyl ou diarylsulfoxyde au déhydrolinalool (produit de départ) s'élève de manière appropriée de 0,2:1 à 1:1, le rapport en poids de l'acide au produit de départ s'élève de manière appropriée de 0,02:1 à 0,1:1, de préférence de 0,04:1 à 0,07:1, en particulier de préférence à 0,05:1, et le rapport en poids du solvant au produit de départ s'élève de 5:1 à 15:1, de préférence de 7:1 à 10:1.
